# EUROPEAN PATENT SPECIFICATION

(11) **EP 1 487 498 B1**
(45) Date of publication and mention of the grant of the patent: **20.05.2009**
(21) Application number: 03709792.0
(22) Date of filing: 17.03.2003
(51) Int. Cl.: A61K 47/36, A61P 27/00

(54) **TOPICAL COMPOSITION COMPRISING A CYCLOFRUCTAN, A CARRIER AND A DRUG**
TOPISCHE ZUSAMMENSETZUNG ENTHALTEND EIN CYCLOFRUCTAN, EINEN TRÄGER UND EINEN ARZNEISTOFF
COMPOSITION TOPIQUE CONTENANT UN CYCLOFRUCTANE, UN EXCIPIENT ET UN PRINCIPE ACTIF

(30) Priority: 18.03.2002 EP 02006085; 12.12.2002 GB 0229019; 23.12.2002 GB 0230033
(43) Date of publication of application: 22.12.2004
(73) Proprietor: NOVARTIS AG, 4056 Basel (CH); Novartis Pharma GmbH, 1230 Wien (AT)
(72) Inventor: KIS, György Lajos, CH-8273 Triboltingen (CH); SCHOCH, Christian, CH-4132 Muttenz (CH); LOHMANN, Dieter, CH-4142 Münchenstein (CH)
(74) Representative: de Weerd, Petrus G.W.
(86) International application number: PCT/EP2003/002760
(87) International publication number: WO 2003/077952

(56) References cited:
- EP-A- 1 273 286
- WO-A-97/10805
- PATENT ABSTRACTS OF JAPAN vol. 1995, no. 05, 30 June 1995 (1995-06-30) -& JP 07 053347 A (MITSUBISHI KASEI CORP), 28 February 1995 (1995-02-28)
- PATENT ABSTRACTS OF JAPAN vol. 018, no. 448 (C-1240), 22 August 1994 (1994-08-22) -& JP 06 141856 A (MITSUBISHI KASEI CORP), 24 May 1994 (1994-05-24)
- PATENT ABSTRACTS OF JAPAN vol. 018, no. 448 (C-1240), 22 August 1994 (1994-08-22) -& JP 06 141879 A (MITSUBISHI KASEI CORP), 24 May 1994 (1994-05-24)
- IMMEL S ET AL: "Cyclofructins with six to ten beta-(12)-linked fructofuranose units: Geometries, electrostatic profiles, lipophilicity patterns, and potential for inclusion complexation" CARBOHYDRATE RESEARCH, ELSEVIER SCIENTIFIC PUBLISHING COMPANY. AMSTERDAM, NL, vol. 313, no. 2, December 1998 (1998-12), pages 91-105, XP004155558 ISSN: 0008-6215
- RANTA V.P. ET AL: 'Ocular Pharmacokonetic...' PHARM RESEARCH vol. 20, no. 9, 2003, pages 1409 - 1416

## Description

The present invention relates especially to a topical ophthalmic drug delivery system comprising a cyclofructan, a drug and at least one carrier.

In WO9710805 a pharmaceutical composition, in particular a preserved ophthalmic composition, comprising a cyclodextrin, a quaternary ammonium salt, an alkylene glycol and a pharmaceutically active compound is described.

A problem associated with topical administration of a drug is the drug permeability through tissue and the topical tolerability. Typically, for ocular and mucus tissue, permeability and tolerability play a significant role both with respect to the active and inactive ingredients. This problem is now surprisingly solved by providing a drug delivery system comprising a cyclofructan (CFR), a drug and at least one carrier selected from a bioerodible polymer and a bioadhesive polymer. CFR in a composition as described hereinafter provides an enhanced drug permeability through tissue, in particular in ocular and mucus tissue.

Since a drug is often washed off from ocular and mucus tissue, this additional problem is addressed in the present invention. This is now solved by providing a drug delivery system which enables sustained and prolonged drug delivery, which drug delivery system contains a polymeric carrier selected from a bioerodible polymer and/or a bioadhesive polymer.

Accordingly, the present invention pertains to a composition comprising a cyclofructan (CFR) of formula I, a drug and at least one carrier selected from a bioerodible polymer and a bioadhesive polymer. As used herein, such a composition represents a drug delivery system.

Cyclofructan (CFR) is known in conjunction with medicinal products and cosmetics. JP 5310805 (Mitsubishi) describes the use of CFR in a pharmaceutical preparation providing a clathrate function. Similar, JP 6298807 (Mitsubishi) describes the use of CFR to increase the solubility of a pharmaceutically effective drug. In JP7053347 a skin cosmetic containing a cyclic inulooligosaccharide as the active component is described. In EP1273286 a composition for retaining active ingredients in personal care compositions is described, optionally comprising cyclic oligosaccharides having a hydrophobic cavity such as cycloinulohexose, cycloinuloheptose, and cycloinuloctose. In JP6141856 a fermentative process for the production of oligosaccharides, which are said to be useful as inclusive agents, is disclosed. In JP6141879 the separation of a cyclic inulooligosacchaide from a saccharide liquid is described. In Immel S, et al, CARBOHYDRATE RESEARCH. Vol: 313, Nr. 2, Page(s): 91-105, 1998, cyclofructins with six to ten beta-(12)-linked fructofuranose units, their geometries, electrostatic profits, lipophilicity patterns, and their potential for inclusion complexation is described.

In a first aspect the present invention discloses the use of a CFR to enhance drug permeation through tissue, and to the use of CFR to enhance drug penetration into tissue, wherein said tissue is preferably selected from ocular tissue and mucus tissue, and wherein said drug is typically administered topically to said tissue. Said use is preferably within the context of the manufacture of a drug delivery system which contains said CFR, further being tailor-made for a disease being preferably treatable by topical treatment.

A first embodiment of the invention thus relates to a composition suitable for topical adminstration to ocular tissue comprising a CFR, a polymeric carrier and a pharmaceutically effective ophthalmic drug, wherein said CFR is a compound of formula I.

In a further embodiment, the invention relates to the use of a CFR in the manufacture of a topical ophthalmic medicament, wherein said medicament comprises a CFR, a carrier and an ophthalmic drug, wherein said CFR is a compound of formula I.

Cyclofructan contains fructose units being connected by beta-(1 → 2) linkages and may be depicted e.g. in formula I, 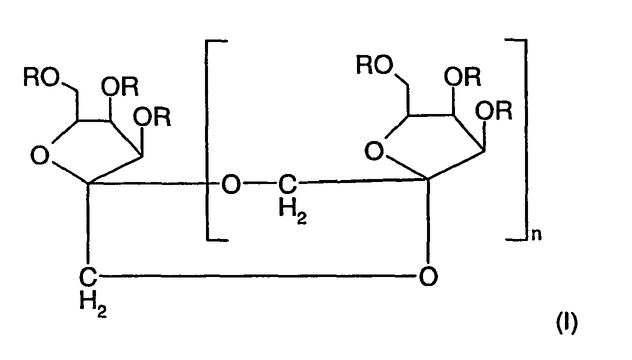 wherein n is from 5 -11,
R is independently from each other H, alkyl, hydroxyalkyl, aminoalkyl, carboxyalkyl, alkoxyalkylen-carbonyl, alkylcarbonyl, alkylcarbamoyl, R'₃Si, or R is a saccharide minus the hydrogen atom from a hydroxy group,
wherein R' denotes independently of each other alkyl and phenyl, preferably methyl and phenyl, more preferably methyl.

In an above CFR, at least one R shall denote H.

Cyclofructan (CFR) is typically consisting of 6 to 12 fructose units, preferably 6 -10 units, also preferably 6 - 8 units, mixtures of 6, 7 and 8 units, most preferably 6 and 7 units. As used herein, the number of fructose units in a CFR are definded by the number directly following the three letters CFR, e.g. 6 fructose units in a cyclofructan shall be depicted as CFR6, 7 units as CFR7, and so on.

The degree of substitution in a CFR of formula I is typically described as a percentage of substitution and refers to the percentage of being different from H. An R being different from H is typically randomly distributed. The degree of substitution is generally from 5 - 99.5%, preferably 5 - 90%, more preferably from 10 - 50%, also preferably from 12 - 45%, and in particular from 15 - 30%. Full substitution with R being different from H (100%) may be very difficult to obtain.

In a preferred aspect R is methyl and the degree of substitution is from 5 - 99.5%, and shall also preferably refer to entirely methylated CFR.

In a highly preferred aspect all R denote H (degree of substitution is 0%).

Alkyl has up to 20 carbon atoms and may be linear or branched. Suitable examples include dodecyl, octyl, hexyl, pentyl, butyl, propyl, ethyl, methyl, 2-propyl, 2-butyl and 3-pentyl. In a preferred definition, alkyl has up to 12 C-atoms and more preferably up to 6 C-atoms. Preferred examples are butyl, propyl, ethyl, and methyl, more preferably ethyl, and methyl, most preferably methyl.

Alkoxy has up to 20 carbon atoms and may be linear or branched. Alkoxy has preferably up to 12 carbon atoms, in particular up to 6 carbon atoms and is, for example, methoxy, ethoxy, propoxy, butoxy, tert-butoxy or hexyloxy.

Preferred examples are methoxy, ethoxy, propoxy, butoxy, more preferably methoxy, ethoxy, in particular methoxy.

Aminoalkyl may be linear or branched and has up to 20 carbon atoms, preferably up to 12 carbon atoms, and in particular 2 to 6 carbon atoms. Examples for aminoalkyl are aminomethyl, aminoethyl, aminopropyl, aminobutyl, aminopentyl, aminohoxyl, aminooctyl or aminodecyl.

Preferred examples are aminoethyl, aminopropyl, aminobutyl, aminopentyl, and aminohexyl. An amino group may additionally be substituted by one or two alkyl group, e.g. for aminoethyl, an addressed substitution may read as N-methylaminoethyl, or N,N-dimethylaminoethyl.

Hydroxyalkyl may be linear or branched and has up to 20 carbon atoms, preferably up to 12 carbon atoms, and in particular 2 to 6 carbon atoms. Examples for hydroxyalkyl are hydroxymethyl, hydroxyethyl, hydroxypropyl, hydroxybutyl, hydroxyhexyl, hydroxyoctyl or hydroxydecyl.

Preferred examples are hydroxyethyl, hydroxypropyl, hydroxybutyl, and hydroxyhexyl.

Alkylene has up to 20 carbon atoms and may be linear or branched. Suitable examples include decylene, octylene, hexylene, pentylene, butylene, propylene, ethylene, methylene, 2-propylene, 2-butylene and 3-pentylene. In a preferred definition, alkylene has up to 12 C-atoms and more preferably up to 6 C-atoms.

Carboxyalkyl may be linear or branched and has up to 20 carbon atoms, preferably up to 12 carbon atoms, and in particular up to 6 carbon atoms. Preferred examples are carboxymethyl, carboxyethyl, carboxypropyl, in particular carboxymethyl.

Alkoxyalkylen-carbonyl may be linear or branched and has up to 20 carbon atoms, preferably up to 12 carbon atoms, and in particular up to 6 carbon atoms. Preferred examples are methoxymethyten carbonyl, methoxyethlyen carbonyl, methoxypropylen carbonyl, ethoxymethlyen carbonyl, ethoxyethylen carbonyl, in particular methoxymethylen carbonyl and methoxyethlyen carbonyl.

Alkylcarbonyl may be linear or branched and has up to 20 carbon atoms, preferably up to 12 carbon atoms, and in particular up to 6 carbon atoms. Preferred examples are methlycarbonyl, ethylcarbonyl, and propylcarbonyl, in particular ethylcarbonyl and methylcarbonyl.

Alkylcarbamoyl may be linear or branched and has up to 20 carbon atoms, preferably up to 12 carbon atoms, and in particular up to 6 carbon atoms. Preferred examples are methylcarbamoyl, ethylcarbamoyl, and propylcarbamoyl, more preferably ethylcarbamoyl.

As used herein, a saccharide shall mean a monosaccharide, disaccharide or trisaccharide.

A monosacharide is understood to be an aldopentose, aldohexose, aldotetrose, ketopentose or ketohexose.

Examples of an aldopentose are D-ribose, D-arabinose, D-xylose and D-lyose; examples of an aldohexose are D-allose, D-altrose, D-glucose, D-mannose, D-gulose, D-idose, D-galactose, D-talose, L-fucose and L-rhamnose; examples of a ketopentose are D-ribulose and D-xylulose; examples of a tetrose are D-erythrose and threose; and examples of a ketohexose are D-psicose. D-fructose, D-sorbose and D-tagatose.

A preferred monosaccharide residue is for example fructosyl, glucosyl, mannosyl or mixtures thereof.

Examples of a disaccharide are trehalose, maltose, isomaltose, cellobiose, gentiobiose, saccharose, lactose, chitobiose, N,N-diacetylchitobiose, palatinose and sucrose. Maltose is a preferred disaccharide.

Raffinose, panose and maltotriose may be mentioned as examples of a trisaccharide. Panosyl is a preferred trisaccharide residue.

As used herein, preferred substituents R in a compound of formula (I) are H, alkyl and hydroxylakyl substituents, more preferred is H, alkyl with up to 6 carbon atoms and hydroxyalkyl with up to 6 carbon atoms, even more preferred is H, methyl and hydroxypropyl, highly preferred is H and methyl, even more preferred is H.

Another preferred R substituent R is selected from H and a monosaccharide, more particular H, fructosyl, and glucosyl.

A preferred cyclofructan ring size is CFR 6, CFR7, CFR8, and mixtures of CFR6,7,8. In an example of a mixed cyclofructan, a CFR 6,7,8, contains 75% CFR6, 20% CFR7 and 5% CFR8 of total weight percent cyclofructan. An above preferred CFR is further preferably CFR6, CFR7, CFR8, and mixtures of CFR6,7,8, wherein R denotes H (degree of substitution is 0%).

As used herein, a drug is in particular selected from the group consisting of:
- Anti-angiogenic drugs, such as VEGF-inhibitors, PKC-inhibitors and the like, e.g. N-benzoylstaurosporine, 1-(3-Chloroanilino)-4-(4-pyridylmethyl)phthalazine,
- Anti-inflammatory drugs, such as steroids, e.g. dexamethasone, fluorometholone , hydrocortisone, prednisolone; or so-called non-steroidal anti-inflammatory drugs (NSAID) such as COX-inhibitors, e.g. diclofenac, valdecoxib, lumiracoxib, ketorolac, or indomethacin;
- Anti-allergic drugs, selected e.g. from FK506, 33-epi-chloro-33-desoxy-ascomycin, cromolyn, emadine, ketotifen, levocabastine, lodoxamide, norketotifen, olopatadine, and rizabene;
- Drugs to treat glaucoma (in particular intraocular pressure treatment), selected e.g. from latanoprost, 15-keto-fatanoprost, unoprostone isopropyl, betaxolol, clonidine, levobunolol and timolol;
- Anti-infective drugs, e.g. selected from ciprofloxacin, chloramphenicol, chlortetracycline, gentamycin, lomefloxacin, neomycin, ofloxacin, polymyxin B and tobramycin;
- Antifungal drugs, e.g. selected from amphotericin B, fluconazole and natamycin;
- Anti-viral drugs such as acyclovir, fomivirsen, ganciclovir, and trifluridine;
- Anesthetic drugs, e.g. selected from cocaine hydrochloride, lidocaine, oxybuprocaine and tetracaine hydrochloride;
- Myopia preventing/inhibiting drugs such as pirenzepine, atropine and the like;
- Miotics, e.g. selected from carbachol, pilocarpine and physostigmine;
- Carbonic anhydrase inhibitors, e.g. selected from acetazolamide and dorzolamide;
- Alpha blocking agents, e.g. selected from apraclonidine and brimonidine; and
- Antioxidants and/or vitamins, e.g. selected from ascorbic acid, α-tocopherol, α-tocopherol acetate, retinol, retinol acetate, and retinol palmitate.

Preferred drugs are selected from:
Anti-angiogenic drugs, anti-inflammatory drugs, anti-allergic drugs, drugs to treat glaucoma, and myopia preventing/inhibiting drugs.

Further preferred are anti-angiogenic drugs, anti-inflammatory drugs, anti-allergic drugs, drugs to treat glaucoma, anti-infective drugs, anti-fungal drugs, anti-viral drugs, anesthetic drugs, myopia preventing/inhibiting drugs, miotics, carbonic anhydrase inhibitors, alpha blocking agents antioxidants and/or vitamins.

In another aspect, and depending on the polymeric carrier, the drug delivery system of the present invention may at room temperature (approximately 22-25°C) be in a solid state, and is in particular selected from a tablet, a film, a rod, a bar, a capsule, a corneal shield, a corneal ring, an implant, an insert, an intra-ocular lens, a therapeutic contact lens, a mini tablet, a mini-disc, and a pellet. Preferably said drug delivery system is selected from a rod, a bar, a capsule, a corneal shield, a corneal ring, an implant, an insert, an intra-ocular lens, a therapeutic contact lens, and a mini-disc, and even more preferred from a corneal shield, a corneal ring, an implant, an insert, an intra-ocular lens, a therapeutic contact lens, and a mini-disc.

As used herein, polymeric carriers suitable for drug delivery systems are for example selected from
- a matrix of a bioerodible polymer being selected from the group consisting of polyhydroxy-acids, such as polylactic acid and polyglycolic acid; polyesters, polyorthoesters, polyanhydrides, polycyanoacrylates, natural gums, such as acacia gum and arabic gum; celluloses, such as carboxymethylcellulose; methacrylate (co)polymers such as Eudragits, e.g. Eudragit RL PO, Eudragit RS PO; and/or
- a bioadhesive polymer being selected from the group consisting of maltodextrin, celluloses, such as carboxymethyl cellulose, hydroxyethyl cellulose; chitosans; hyaluronic acid; polyacrylates e.g. carbopol; polycarbophils e.g. Noveon AA-1; polyvinylalcohol such as Mowiol 26-88; polyvinylpyrrolidone such as povidone K30.

The amount of a polymeric carrier used in a composition or a drug delivery system of the present invention is in the range of from 0.01 to approximately 99% by weight, preferably in the range of from 1 - 95% by weight, more preferably in the range of from 10 - 90% by weight, even more preferably in the range of from 15 - 85% by weight, and in the range of from 20 - 80% by weight.

The use of a drug in conjunction with a polymeric carrier and a cyclofructan provides typically a synergistic advantage of sustained drug delivery with improved drug permeability.

Accordingly, a further aspect is a drug delivery system which comprises a polymeric carrier being selected from:
A matrix of a bioerodible polymer being selected from the group consisting of polyhydroxy-acids, such as polylactic acid and polyglycolic acid; polyesters, polyorthoesters, polyanhydrides, polycyanoacrylates, natural gums, such as acacia gum and arabic gum; celluloses, such as carboxymethylcellulose; methacrylate (co)polymers such as Eudragits, e.g. Eudragit RL PO, Eudragit RS PO; and/or
A bioadhesive polymer being selected from the group consisting of maltodextrin, celluloses, such as carboxymethyl cellulose, hydroxyethyl cellulose; a cyclodextrin, chitosans; hyaluronic acid; polyacrylates e.g. carbopol; polycarbophils e.g. Noveon AA-1; polyvinylalcohol such as Mowiol 26-88; polyvinylpyrrolidone such as povidone K30:
a cyclofructan, and
a pharmaceutically effective drug.

Additional carriers might be used in the manufacture of a drug delivery system, for example by adapting said system to specific needs, e.g. ophthalmically acceptable issues, and are for example water, mixtures of water and water-miscible solvents, such as C₁- to C₇-alkanols, vegetable oils or mineral oils comprising from 0.5 to 5% by weight hydroxyethylcellulose, ethyl oleate, carboxymethyl-cellulose, polyvinyl-pyrrolidone and other non-toxic water-soluble polymers for ophthalmic uses, such as, for example, acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as polyvinyl methyl ether, polyethylene oxide or mixtures of those polymers.

The concentration of an above carrier is, for example, from 1 to 100000 times the concentration of the active ingredient.

The drug delivery system of the present invention may further comprise a tonicity enhancing agent.

Tonicity enhancing agents are, for example, ionic compounds, such as alkali metal or alkaline earth metal halides, such as, for example, CaCl₂, KBr, KCI, LiCl, Nal, NaBr or NaCl, or boric acid. Non-ionic tonicity enhancing agents are, for example, urea, glycerol, sorbitol, mannitol, propylene glycol, or dextrose. For example, sufficient tonicity enhancing agent is added to impart an osmolality of approximately from 50 to 1000 mOsmol.

For the adjustment of the pH, preferably to a physiological pH, buffers may especially be useful. Examples of buffer substances are acetate, ascorbate, borate, hydrogen carbonate /carbonate, citrate, gluconate, lactate, phosphate, propionate and TRIS (tromethamine) buffers. Tromethamine and borate buffer are preferred buffers. The amount of buffer substance added is, typically, that necessary to ensure and maintain a physiologically tolerable pH range. The pH range is generally in the range of from 4 to 9, preferably from 4.5 to 8.5 and more preferably from 5.0 to 8.2.

The drug delivery system of the present invention may further comprise a preservative, e.g on storage or to inhibit microbial growth.

A preservative may typically be selected from a quaternary ammonium compound such as benzalkonium chloride, benzoxonium chloride or the like. Benzalkonium chloride is better described as: N-benzyl-N-(C₈-C₁₈alkyl)-N,N-dimethylammonium chloride. Examples of preservatives different from quaternary ammonium salts are alkyl-mercury salts of thiosalicylic acid, such as, for example, thiomersal, phenylmercuric nitrate, phenylmercuric acetate or phenylmercuric borate, parabens, such as, for example, methylparaben or propylparaben, alcohols, such as, for example, chlorobutanol, benzyl alcohol or phenyl ethanol, guanidine derivatives, such as, for example, chlorohexidine or polyhexamethylene biguanide, sodium perborate, Germal^{®}II or sorbic acid. Preferred preservatives are quaternary ammonium compounds, in particular benzalkonium chloride, alkyl-mercury salts and parabens. Where appropriate, a sufficient amount of preservative is added to ensure protection against secondary contaminations during use caused by bacteria and fungi.

A drug delivery system of the present invention may additionally require the presence of a solubilizer, in particular if the active or the inactive ingredients or the active and intended inactive ingredients tend to form a suspension or an emulsion.

A solubilizer suitable for compositions of the invention is for example selected from the group consisting of tyloxapol, fatty acid glycerol polyethylene glycol esters, fatty acid polyethylene glycol esters, polyethylene glycols, glycerol ethers, a cyclodextrin (for example α-, β- or γ-cyclodextrin, e.g. alkylated, hydroxyalkylated, carboxyalkylated or alkyloxycarbonyl-alkylated derivatives, or mono- or diglycosyl-α-, β- or γ- cyclodextrin, mono- or dimaltosyl-α-, β- or γ-cyclodextrin or panosyl-cyclodextrin), polysorbate 20, polysorbate 80 or mixtures of those compounds. A specific example of an especially preferred solubilizer is a reaction product of castor oil and ethylene oxide, for example the commercial products Cremophor EL <!Ðor Cremophor RH 40^{®}. Reaction products of castor oil and ethylene oxide appear to be particularly good solubilizers that are tolerated extremely well by the eye. Another preferred solubilizer is selected from tyloxapol and from a cyclodextrin. The concentration used depends especially on the concentration of the active ingredient. The amount added is typically sufficient to solubilize the active ingredient. For example, the concentration of the solubilizer is typically from 0.1 to 5000 times the concentration of the active ingredient.

Further excipients may be comprised in a drug delivery system of the invention, which may in particular function as a combined stabilizer/solubilizer. Such a combined additional stabilizer/solubilizer is for example a cyclodextrin. A preferred cyclodextrin is in particular selected from the group of α-cyclodextrin, β-cyclodextrin, methyl-β-cyclodextrin, methyl-γ-cyclodextrin, γ-cyclodextrin, hydroxypropyl-β-cyclodextrin, hydroxypropyl-γ-cyclodextrin, dimethyl-β- cyclodextrin and dimethyl-γ-cyclodextrin. The amount is generally in the range of from approximately 0.01 to approximately 90% by weight, more preferably in the range of from 0.1 - 20% by weight.

A drug delivery system of the invention may comprise further non-toxic excipients, such as, for example, emulsifiers, wetting agents or fillers, such as, for example, the polyethylene glycols having an average molecular weight of 200, 300, 400 and 600, or higher polyethylene glycols, also called Carbowax being designated Carbowax 1000, 1500, 4000, 6000 and 10000. Other excipients that may be used if desired are listed below but they are not intended to limit in any way the scope of the possible excipients. They are especially complexing agents, such as disodium-EDTA or EDTA, antioxidants, such as ascorbic acid, acetyloysteine, cysteine, sodium hydrogen sulfite, butyl-hydroxyanisole, butyl-hydroxytoluene or alpha-tocopherol acetate; stabilizers, such thiourea, thiosorbitol, sodium dioctyl sulfosuccinate or monothioglycerol: or other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from approximately 0.0001 to approximately 90% by weight.

The amount of CFR present in a drug delivery system of the present invention generally depends on the drug being used and is typically in the range of 0.1 - 35%, preferably from 0.5 - 25%, more preferably from 5 - 10%, and 15 - 20%, also preferably from 0.1 - 5%, 0.5 - 5% and 1-5% by total weight of a corresponding pharmaceutical composition.

Also preferred are the amount and type CFR as described in the working examples.

The invention further discloses a method of improving drug permeability through ocular tissue, which method comprises the steps of:
Conventionally admixing an effective amount of a CFR of formula I, an effective amount of a drug, and at least a polymeric carrier;
optionally admixing one or more further ingredients selected from the group of buffers, tonicity enhancing agents, preservatives, solubilizers, stabilizers/solubilizers, and complexing agents;
optionally forming the above admixed components in a mold:
and
administering said admixture comprising said CFR and said drug to said tissue in need of drug treatment.

Said tissue is preferably in the ocular tissue, such as corneal epithelial cells, and conjunctival cells.

The improvement of drug permeation, for example in the eye, typically provides the benefit of improved tolerability and/or improved efficacy, typically in a synergistic fashion, because CFR of formula I appears to improve the bio-availability of a corresponding drug. Therefore, typically less drug is needed for obtaining a comparable pharmacological efficacy as obtained by a composition not comprising a CFR of formula I. Also, typically the onset of action of a CFR of formula I-formulated composition, e.g. upon topical ophthalmic administration, appears to be Improved.

The invention also relates to the use of a cyclofructan of formula I in the manufacture of a solid state, topical, ophthalmic medicament for the treatment of an ocular disease. Said solid state medicament comprises a CFR of formula I, a polymeric carrier suitable for solid state medicaments and a pharmaceutically effective drug.

Further disclosed herein is a method of improving drug permeability through, and drug penetration into tissue, wherein said tissue is selected from ocular tissue and wherein said drug is administered topically to said tissue, which method comprises a CFR which is a compound of formula I.

A preferred embodiment is related to an ophthalmic composition comprising a CFR of formula I, an ophthalmically acceptable polymeric carrier and an ophthalmic drug, said polymeric carrier being for example selected from cellulose derivatives, hyaluronic acid, cyclodextrins, polyvinylalcohol, polyvinylpyrrolidone, neutral Carbopol, or mixtures thereof -

The use of a CFR of formula I in the context with enhanced drug permeation trough tissue upon topical administration is not conditional to a special polymeric carrier, as e.g. described above. Accordingly, as used herein a carrier represents any other carrier if compatible with topical administration.

More preferably it pertains to the use of a cyclofructan of formula I in the manufacture of a topical ophthalmic medicament, wherein said medicament comprises a CFR of formula I, a carrier, preferably a polymeric carrier and an ophthalmic drug.

### Example: Increase of corneal permeation

### Corneal permeation system:

The system used was a modified Valia-Chlen system consisting of two water-jacketed cells for temperature control. Each cell was filled with GBR buffer (see below), stirred by a magnet and continuously gassed with Oxycarbon (5 % CO₂ / 95 % O₂). During an experiment, the cells were separated by the cornea, one cell containing the test substance dissolved in GBR and acting as donor (tear side), the other one being the acceptor (aqueous humor side).

### Corneas:

Pig eyes were obtained from the local abattoir. They were kept in Dulbecco's MEM (minimal essential medium) with Glutamax-I (Gibco) on Ice and used within a few hours after receipt.

### Buffers:

Buffers for *in vitro* corneal permeation studies were adapted from glutathione-bicarbonate-Ringer (GBR) solution. "GBR aqueous humor" was used in the acceptor cell and "GBR tears" on the donor side for equilibration. Their composition is listed in Table 1.

### Assay of corneal permeation:

On receipt from the abattoir the eye was mounted on a dissection board, cornea facing up. After checking integrity of the cornea, the sclera was incised approximately 1-2 mm from the corneal rim with a scalpel and the anterior segment was excised. The iris and lens were carefully removed with forceps without damaging the corneal structures. The cornea was then mounted between the two cells of the permeation system with the help of a pinch clamp. Immediately, 3 ml of prewarmed and gassed GBR buffer were added to each cell, carefully removing any trapped air bubbles in the cells. The system was gassed and stirred for about 30 minutes at 35° C. After equilibration, the donor side was emptied and the same amount of prewarmed formulation of active substance was added at time t=0. An aliquot of 300 µl "GBR aqueous humor" was taken at time t=0 from the acceptor cell and the missing volume was replaced by the same volume of fresh buffer. Subsequently, this procedure was repeated in the acceptor cell at predefined time points and the aliquots were analysed for active by HPLC. Both compartments were kept under constant stirring with small magnets. The usual duration of an experiment was 180 minutes which was also the time of contact of the formulation with the cornea.

**Table 1: Buffers used for in vitro corneal permeation experiments**

| Constituent | GBR aqueous humor | GBR tears |
|---|---|---|
| | Concentration [mM] | Concentration [mM] |
| NaCl | 95.75 | 115.75 |
| NaH₂PO₄ | 1.25 | 1.25 |
| KCl | 4 | 20 |
| CaCl₂ | 2 | 2 |
| MgCl₂ | 1 | 1 |
| Adenosine | 0.5 | 0.5 |
| NaHCO₃ | 23 | 23 |
| Glutathione reduced | 0.3 | 0.3 |
| Glucose | 77.7 | 27.75 |
| H₂O | q.s. | q.s. |
| pH | 7.3-7.4* | 7.3-7.4* |
| Osmolality | 297 mOsm/kg | 311 mOsm/kg |

| | | |
|---|---|---|
| * when gassed with 5% CO₂ / 95% O₂ | | |

### Corneal permeation experiments with diclofenac formulations

### 1) Diclofenac sodium 0.1% without thiomersal (marketed Voltaren Ophtha formulation, SDU)

| Time (min) | Average permeated amount (micro-gram) | S.D. |
|---|---|---|
| 0 | 0 | 0 |
| 30 | 0 | 0 |
| 60 | 0 | 0 |
| 90 | 0.215981 | 0.196208 |
| 120 | 0.689412 | 0.418657 |
| 180 | 1.979619 | 0.878349 |

### 2) Diclofenac sodium 0.1% with 2% HP-gamma-CD and without BAC

| Time (min) | Average permeated amount (micro-gram) | S.D. |
|---|---|---|
| 0 | 0 | 0 |
| 30 | 0 | 0 |
| 60 | 0.060704 | 0.105143 |
| 90 | 0.870969 | 0.348648 |
| 120 | 1.794925 | 0.553737 |
| 180 | 5.321767 | 1.036315 |

### 3) Diclofenac sodium 0.1% with 2% CFR6 and without BAC

| Time (min) | Average permeated amount (micro-gram) | S.D. |
|---|---|---|
| 0 | 0 | 0 |
| 30 | 0 | 0 |
| 60 | 2.092435 | 0.781957 |
| 90 | 6.113682 | 1.163991 |
| 120 | 11.19923 | 1.621378 |
| 180 | 21.59050 | 3.123698 |

| | | |
|---|---|---|
| BAC = benzalkonium chloride HP-gamma-CD = hydroxypropyl-γ-cyclodextrin CFR6 = circular (or cyclic) hexameric cyclofructan, 6 fructose units (R=H, 0% substitution) | | |

In the above experiments [item 2) & item 3)] the efficacy in drug permeation is directly comparable with respect to the prior art situation (HP-gamma-CD) and an embodiment of this invention, namely CFR6.

## Claims

1. Use of a CFR in the manufacture of a topical ophthalmic medicament, wherein said medicament comprises a CFR, a carrier and an ophthalmic drug, and wherein said CFR is a compound of formula I, 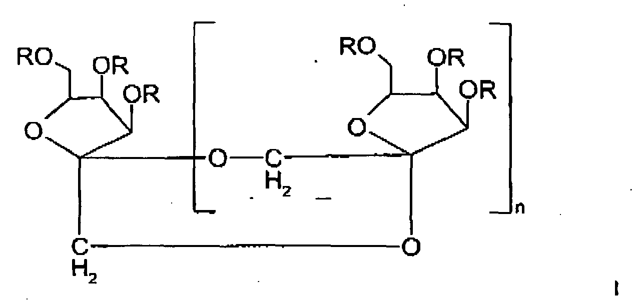 wherein
n is from 5 to 11,
R is independently from each other H, alkyl, hydroxyalkyl, aminoalkyl, carboxyalkyl, alkoxyalkylen-carbonyl, alkylcarbonyl, alkylcarbamoyl, R'₃Si, or
R is a saccharide minus the hydrogen atom from a hydroxy group, and
R' denotes independently of each other alkyl and phenyl.

2. A composition suitable for topical administration to ocular tissue, comprising a CFR, a polymeric carrier and a pharmaceutically effective ophthalmic drug, wherein said CFR is a compound of formula I, 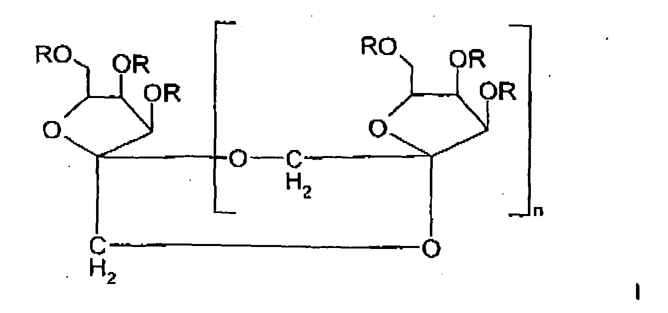 wherein
n is from 5 to 11,
R is independently from each other H, alkyl, hydroxyalkyl, aminoalkyl, carboxyalkyl, alkoxyalkylen-carbonyl, alkylcarbonyl, alkylcarbamoyl, R'₃Si, or
R is a saccharide minus the hydrogen atom from a hydroxy group, and
R' denotes independently of each other alkyl and phenyl.

3. The use or a composition according to any one of claims 1 or 2, wherein R in formula I is H.

4. The use or a composition according to any one of claims 1 or 2 wherein said CFR is CFR 6, CFR7, CFR8. and mixtures of CFR,6,7,8.

5. The use or a composition according to any one of claims 1 or 2, wherein said drug is selected from anti-angiogenic drugs, anti-inflammatory drugs, anti-allergic drugs, drugs to treat glaucoma, anti-infective drugs, anti-fungal drugs, anti-viral drugs, anesthetic drugs, myopia preventing/inhibiting drugs, miotics, carbonic anhydrase inhibitors, alpha blocking agents antioxidants and/or vitamins.

6. The use or a composition according to any one of claims 1 or 2, wherein said carrier is selected from:
A matrix of a bioerodible polymer being selected from the group consisting of polyhydroxy-acids, polyesters, polyorthoesters, polyanhydrides, polycyanoacrylates, natural gums, celluloses and methacrylate (co)polymers; and/or
A bioadhesive polymer being selected from the group consisting of maltodextrin, celluloses, chitosans, hyaluronic acids; polyacrylates; polycarbophils; polyvinylalcohols and polyvinylpyrrolidones.

7. A composition according to any one of claims 2 to 6, which is at room temperature (approximately 22-25°C) in a solid state.

8. A composition or a medicament according to any one of claims 1, or 2 to 5, which is a drug delivery system.

9. A composition or a medicament according to claim 8, wherein the drug delivery system is selected from the group consisting of a rod, a bar, a capsule, a corneal shield a corneal ring, an implant, an insert, an intra-ocular lens, a therapeutic contact lens, and a mini-disc.

10. A composition or a medicament according to any one of claims 1, or 2 to 9, further comprising one or more of the ingredients selected from the group of buffers, tonicity enhancing agents, preservatives, solubilizers, stabilizers/solubilizers, and complexing agents.

## Patentansprüche

1. Verwendung eines CFR zur Herstellung eines topischen ophthalmischen Arzneimittels, worin dieses Arzneimittel ein CFR, einen Träger und einen ophthalmischen Wirkstoff umfasst, und worin dieses CFR eine Verbindung der folgenden Formel I ist 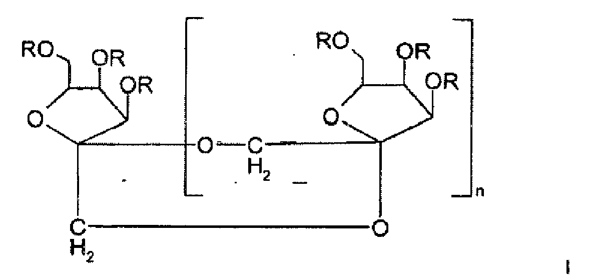 worin
n für 5 bis 11 steht,
R unabhängig voneinander für H, Alkyl, Hydroxylalkyl, Aminoalkyl, Carboxyalkyl, Alkoxyalkylencarbonyl, Alkylcarbonyl, Alkylcarbamoyl oder R'₃Si steht, oder
R für ein Saccharid minus dem Wasserstoff aus einer Hydroxygruppe steht, und
R' unabhängig voneinander für Alkyl oder Phenyl steht.

2. Zusammensetzung, die sich für eine topische Verabreichung an Augengewebe eignet, umfassend ein CFR, einen polymeren Träger und einen pharmazeutisch wirksamen ophthalmisehen Wirkstoff, worin das CFR eine Verbindung der folgenden Formel I ist 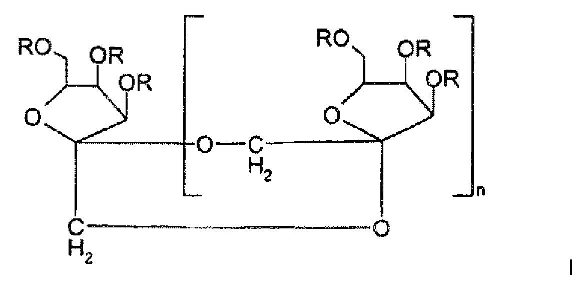 worin
n für 5 bis 11 steht,
R unabhängig voneinander für H, Alkyl, Hydroxyalkyl, Aminoalkyl, Carboxyalkyl, Alkoxyalkylencarbonyl, Alkylcarbonyl, Alkylcarbamoyl oder R'₃Si steht, oder
R für ein Saccharid minus dem Wasserstoff aus einer Hydroxygruppe steht, und
R' unabhängig voneinander für Alkyl oder Phenyl steht.

3. Verwendung oder Zusammensetzung nach einem der Ansprüche 1 oder 2, worin R in der Formel I für H steht.

4. Verwendung oder eine Zusammensetzung nach einem der Ansprüche 1 oder 2, worin CFR für CFR6, CFR7, CFR8 oder Gemische von CFR6, CFR7 und CFR8 steht.

5. Verwendung oder eine Zusammensetzung nach einem der Ansprüche 1 oder 2, worin der Wirkstoff ausgewählt ist aus antiangiogenen Mitteln, antiinflammatorischen Mitteln, antiallergischen Mitteln, Mitteln zur Behandlung von Glaukom, antiinfektiven Mitteln, antifungalen Mitteln, antiviralen Mitteln, anästhetischen Mitteln, Mitteln zur Prävention oder Inhibition von Myopie, Miotika, Carboanhydraseinhibitoren, alpha-Blockern, Antioxidantien und/oder Vitaminen.

6. Verwendung oder eine Zusammensetzung nach einem der Ansprüche 1 oder 2, worin der Träger ausgewählt ist aus:
einer Matrix aus einem bioerodierbaren Polymer, das ausgewählt ist aus der Gruppe, die besteht aus Polyhydroxysäuren, Polyestern, Polyorthoestern, Polyanhydriden, Polycyanoacrylaten, natürlichen Gummis, Cellulosen und Methacrylat(co)polymeren, und/oder
einem bioadhäsiven Polymer, das ausgewählt ist aus der Gruppe, die besteht aus Maltodextrin, Cellulosen, Chitosanen, Hyaluronsäuren, Polyacrylaten, Polycarbophilen, Polyvinylalkoholen und Polyvinylpyrrolidonen.

7. Zusammensetzung nach einem der Ansprüche 2 bis 6, die bei Raumtemperatur (etwa 22 bis 25 °C) in einem festen Zustand vorliegt.

8. Zusammensetzung oder ein Arzneimittel nach einem der Ansprüche 2 bis 5, die ein Spendersystem für Arzneimittel ist.

9. Zusammensetzung oder ein Arzneimittel nach Anspruch 8, worin das Spendersystem für Arzneimittel ausgewählt ist aus der Gruppe, die besteht aus einem Stab, einem Riegel, einer Kapsel, einem Corneaschutz, einem Cornearing, einem Implantat, einem Einsatz, einer Intraokularlinse, einer therapeutischen Kontaktlinse und einer Minischeibe.

10. Zusammensetzung oder ein Arzneimittel nach einem der Ansprüche 1 oder 2 bis 9, weiter umfassend ein oder mehr Hilfsstoffe, die ausgewählt sind aus der Gruppe von Puffern, Tonizitätsverbesserungsmitteln, Konservierungsmitteln, Solubilisatoren, Stabilisatoren/Solubilisatoren und Komplexbildnern.

## Revendications

1. Utilisation d'un cyclofructane (CFR) dans la préparation d'un médicament ophtalmique topique, ledit médicament comprenant un CFR, un excipient et un agent ophtalmique, ledit CFR étant un composé de formule I suivante: 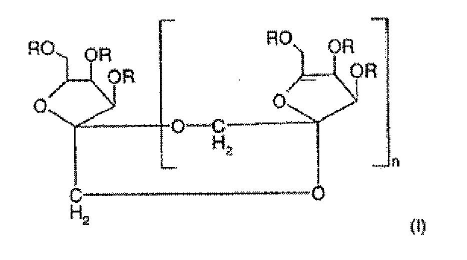 dans laquelle
n est compris entre 5 et 11,
chaque R représente indépendamment H, une groupe alkyle, hydroxyalkyle, aminoalkyle, carboxyalkyle, alcoxyalkylène-carbonyle, alkylcarbonyle ou alkylcarbamoyle, R'₃Si, ou un saccharide moins l'atome d'hydrogène provenant d'un groupe hydroxy, et
chaque R' représente indépendamment un groupe alkyle ou phényle.

2. Composition adaptée à l'application topique sur le tissu oculaire, comprenant un CFR, un excipient polymère et un agent ophtalmique efficace sur le plan pharmaceutique, ledit CFR étant un composé de formule I suiwante : 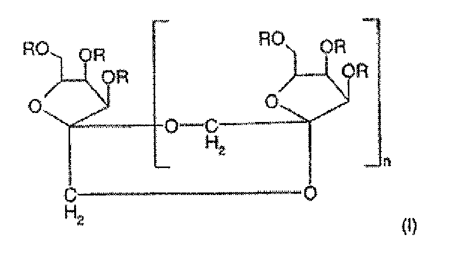 dans laquelle
n est compris entre 5 et 11,
chaque R représente indépendamment H, un groupe alkyle, hydroxyalkyle, aminoalkyle, carboxyalkyle, alcoxyalkylène-carbonyle, alkylcarbonyle ou alkylcarbamoyle, R'₃Si, ou un saccharide moins l'atome d'hydrogène provenant d'un groupe hydroxy, et
chaque R' représente indépendamment un groupe alkyle ou phényle.

3. Utilisation ou composition selon la revendication 1 ou 2, dans laquelle R représente H dans la formule 1.

4. Utilisation ou composition selon la revendication 1 ou 2, dans laquelle ledit CFR est un CFR6, un CFR7, un CFR8, ou des mélanges de ceux-ci.

5. Utilisation ou composition selon la revendication 1 ou 2, dans laquelle ledit agent est choisi parmi des agents anti-angiogéniques, des agents anti-inflammatoires, des agents anti-allergiques, des agents pour traiter un glaucome, des agents anti-infectieux, des agents antifongiques, des agents antiviraux, des agents anesthésiques, des agents de prévention/d'inhibition de la myopie, des myotiques, des inhibiteurs de l'anhydrase carbonique, des agents alpha-bloquants, des agents antioxydants et/ou des vitamines.

6. Utilisation ou composition selon la revendication 1 ou 2, dans laquelle ledit excipient est choisi parmi une matrice comprenant un polymère bioérodable choisi dans le groupe constitué par les polyhydroxyacides, les polyesters, les polyorthoesters, les polyanhydrides, les polycyanoacrylates, les gommes naturelles, les celluloses et les (co)polymères de méthacrylate ; et/ou un polymère bioadhésif choisi dans le groupe constitué par les maltodextrines, les celluloses, les chitosanes, les acides hyaluroniques, les polyacrylates, les polycarbophiles, les alcools de polyvinyle et les polyvinylpyrrolidones.

7. Composition selon l'une quelconque des revendications 2 à 6, qui se présente à l'état solide à la température ambiante (approximativement 22 à 25 °C).

8. Composition ou médicament selon l'une quelconque des revendications 1 à 5, qui est un système de libération d'un médicament.

9. Composition ou médicament selon la revendication 8, dans laquelle/lequel le système de libération de médicament est choisi dans le groupe constitué par une tige, une barre, une gélule, une protection cornéenne, un anneau cornéen, un implant, un insert, une lentille intraoculaire, une lentille de contact thérapeutique et une minidisque.

10. Composition ou médicament selon l'une quelconque des revendications 1 à 9, comprenant en outre un ou plusieurs des ingrédients choisis dans le groupe constitué par les tampons, les agents améliorant la tonicité, les conservateurs, les agents solubilisants, les stabilisants/solubilisants et les agents complexants.
